# EUROPEAN PATENT APPLICATION

(11) **EP 4 582 536 A1**
(43) Date of publication of application: **09.07.2025**
(21) Application number: 23859849.4
(22) Date of filing: 07.07.2023
(51) Int. Cl.: C12N 5/10, C12N 5/071, C12P 1/00, C12P 21/08, G16B 40/00

(54) **METHOD FOR PREDICTING PRODUCTION STABILITY OF CLONE THAT PRODUCES USEFUL SUBSTANCE, INFORMATION PROCESSING DEVICE, PROGRAM, AND PREDICTION MODEL GENERATION METHOD**

(30) Priority: 31.08.2022 JP 2022138789
(71) Applicant: FUJIFILM Corporation, Tokyo 106-8620 (JP)
(72) Inventor: UMEKAWA, Masao, Ashigarakami-gun, Kanagawa 258-8577 (JP); SUZUKI, Takafumi, Ashigarakami-gun, Kanagawa 258-8577 (JP); SATO, Masahiro, Ashigarakami-gun, Kanagawa 258-8577 (JP); NAGASE, Masaya, Ashigarakami-gun, Kanagawa 258-8577 (JP); MATSUURA, Tatsuya, Ashigarakami-gun, Kanagawa 258-8577 (JP); MURAKAMI, Yuta, Ashigarakami-gun, Kanagawa 258-8577 (JP)
(74) Representative: Hoffmann Eitle
(86) International application number: PCT/JP2023/025263
(87) International publication number: WO 2024/048079

(57) **Abstract**

Provided are a method, an information processing apparatus, a program, and a prediction model generation method that can predict production stability of a clone that produces a useful substance with high accuracy and at a low cost. One or more processors execute acquiring culture data of one or more types of clones for a clone that produces a useful substance, analyzing the culture data and limiting the clones to a prediction target, and using data measured for a clone as the prediction target to predict production stability of the useful substance by the clone as the prediction target. The production stability may be defined by presence or absence of a change in a production amount of the useful substance between a start of culture and after a predetermined culture period.

## Description

### BACKGROUND OF THE INVENTION

### 1. Field of the Invention

The present disclosure relates to an information processing technique and a machine learning technique for predicting production stability of a clone that produces a useful substance.

### 2. Description of the Related Art

In recent years, industrial applications of a manufacturing method of allowing cells to produce a complex useful substance that is difficult to prepare through conventional chemical synthesis have been advancing. One example thereof is biopharmaceuticals, which account for equal to or greater than half of items in the top 10 global pharmaceutical sales rankings and approximately two-thirds of sales revenue. Biopharmaceuticals utilize complex proteins and the like and are significantly difficult to artificially synthesize chemically, as compared to conventional small molecule drugs. Therefore, for antibody drugs, which are an example of biopharmaceuticals, for example, a production method of inserting a gene corresponding to a desired human protein into Chinese hamster ovary cells (CHO cells) or the like, allowing the cells to produce the desired protein through a cell function, and extracting and purifying the protein to manufacture antibody drugs is widely used.

Since it is not possible to finely control the insertion of the genes into the cells as mentioned above, it is common to insert the genes into a large number of cells at the same time. In this case, considering that insertion positions of the genes in the generated individual cells are random, many regulatory authorities require that the cells responsible for antibody production after gene insertion are derived from a single cell, and their properties do not change through subculture, that is, so-called monoclonality is demanded, in order to stabilize the antibodies as pharmaceuticals and ensure quality.

Therefore, a single cell is extracted from individual cells with random gene insertion positions, the single cell is proliferated to prepare a cell clone (hereinafter, referred to as a clone), and the clone is made to produce antibodies, thereby ensuring monoclonality. The clone of an embodiment of the present invention means a population of genetically identical cells or cells constituting the population.

Meanwhile, in industrialization, there is a demand for a clone having a high-quality antibody production capacity. Here, the high-quality antibody production capacity refers to having a high antibody-producing ability at the present point in time and maintaining a stable antibody-producing ability over a long culture period. As mentioned above, clones prepared from individual cells with random gene insertion positions have variations in the antibody-producing ability, making it necessary to discriminate whether each clone has a high-quality antibody production capacity. Whether or not a clone is a high-producing clone having a high antibody-producing ability at the present point in time can be discriminated through a two-week standard test, but in order to discriminate the production stability of whether or not the antibody-producing ability is stable over a long culture period, experimental verification (stability test) through long-term culture actually lasting about several months is essential.

Under such a background, WO2016/075216A proposes a method of predicting production stability of a recombinant protein of a clone several months later based on gene expression data of the clone obtained at the present point in time. In addition, Uros Jamnikar, Petra Nikolic, Ales Belic, Marjanca Blas, Dominik Gaser, Andrej Francky, Holger Laux, Andrej Blejec, Spela Baebler, and Kristina Gruden, "Transcriptome study and identification of potential marker genes related to the stable expression of recombinant proteins in CHO clones" BMC Biotechnology volume 15, Article number 98 (2015) proposes a method of identifying a marker gene in which stable expression of a recombinant protein can be predicted in an early stage of clone development, and predicting the production stability of the recombinant protein in the early stage of clone development.

### SUMMARY OF THE INVENTION

However, the method described in WO2016/075216A cannot be said to be sufficient in terms of prediction accuracy. Additionally, since genetic analysis or the like for a large number of clones generally requires high costs, there has also been a problem that a cost reduction effect obtained by predicting the production stability of the recombinant protein is offset by increased costs due to genetic analysis or the like for prediction. It is conceivable to narrow down the number of clones that are prediction targets for the production stability in order to reduce costs; however, in this case, the number of clones having high production stability among the prediction targets is also reduced, and as a result, the number of clones having high production stability to be obtained is reduced, making it difficult to simply narrow down the number of clones as the prediction targets.

A first object to be achieved by the present disclosure is to provide means for predicting production stability of a useful substance in a clone with high accuracy. A second object is to provide means for reducing a cost of predicting the production stability of the useful substance in the clone.

The present disclosure has been made in view of such circumstances, and an object of the present disclosure is to provide a method, an information processing apparatus, a program, and a prediction model generation method that can predict production stability of a clone that produces a useful substance with high accuracy and at a low cost.

According to a first aspect of the present disclosure, there is provided a method of predicting production stability of a clone that produces a useful substance, the method comprising: causing one or more processors to execute: acquiring culture data of one or more types of clones; analyzing the culture data and limiting the clones to a prediction target; and using data measured for a clone as the prediction target to predict the production stability of the useful substance by the clone as the prediction target.

According to the first aspect, since the prediction target is limited based on information obtained from the culture data to predict the production stability, the production stability can be predicted with high accuracy as compared to a case in which the target is not limited. In addition, since data necessary for the prediction need only be acquired for the limited clone that is the prediction target, cost reduction is possible.

The production stability to be predicted may represent a future state of a clone several months later, as in the production stability experimentally verified through long-term culture actually lasting several months. For example, the production stability may be evaluated from the viewpoint of whether or not an initial production amount is maintained even after the long-term culture. According to the first aspect, a result of a stability test that requires long-term culture can be predicted with high accuracy and at a low cost.

According to a second aspect of the present disclosure, in the method according to the first aspect, a configuration may be employed in which the production stability is defined by presence or absence of a change in a production amount of the useful substance between a start of culture and after a predetermined culture period.

According to a third aspect of the present disclosure, in the method according to the first or second aspect, a configuration may be employed in which causing the one or more processors to execute setting an index obtained from the culture data and a threshold value related to the index is further provided, and the prediction target is limited based on a value of the index and the threshold value.

According to a fourth aspect of the present disclosure, in the method according to the third aspect, a configuration may be employed in which the threshold value is adjusted such that prediction accuracy of the production stability is higher than prediction accuracy in a case in which the prediction target is not limited.

According to a fifth aspect of the present disclosure, in the method according to the third or fourth aspect, a configuration may be employed in which the threshold value is defined using a ranking of the value of the index. The "ranking" may be a ranking in a case in which the values of the index for a plurality of clones are arranged in descending order, and a ranking in a case in which the values of the index for a plurality of clones are arranged in ascending order. For example, the threshold value may be defined as the top 40% of relative rankings in the population that includes a plurality of clones, or the like.

According to a sixth aspect of the present disclosure, in the method according to any one of the third to fifth aspects, the prediction target may be a top population of the values of the index.

According to a seventh aspect of the present disclosure, in the method according to any one of the third to sixth aspects, the index may be a production amount of the useful substance.

According to an eighth aspect of the present disclosure, in the method according to any one of the third to sixth aspects, the index may be an integral viable cell density.

According to a ninth aspect of the present disclosure, in the method according to any one of the third to sixth aspects, the index may be a lactic acid concentration.

According to a tenth aspect of the present disclosure, in the method according to any one of the first to ninth aspects, a configuration may be employed in which the data used for the prediction of the production stability includes one or more gene expression levels.

According to an eleventh aspect of the present disclosure, in the method according to any one of the first to tenth aspects, a configuration may be employed in which the one or more processors predict the production stability by using a model that receives an input of the data of the prediction target and that performs two-class classification into stable or unstable.

According to a twelfth aspect of the present disclosure, in the method according to the eleventh aspect, the model may be a model that has been trained through machine learning using a plurality of pieces of training data in which the data for a training clone, which is limited in the same manner as the clone as the prediction target, and a ground truth stability label are associated with each other.

According to a thirteenth aspect of the present disclosure, in the method according to the twelfth aspect, a configuration may be employed in which the plurality of pieces of training data include the training data for a plurality of types of clones that produce different useful substances, and the one or more processors predict the production stability for a clone that produces a useful substance different from the useful substance used for the training of the model.

According to a fourteenth aspect of the present disclosure, in the method according to any one of the first to thirteenth aspects, the useful substance may be any of a protein, a peptide, or a virus that is a pharmaceutical raw material.

According to a fifteenth aspect of the present disclosure, in the method according to any one of the first to fourteenth aspects, the useful substance may be an antibody or an antibody-like protein.

According to a sixteenth aspect of the present disclosure, in the method according to any one of the first to fifteenth aspects, the clone may be a vertebrate-derived cell.

According to a seventeenth aspect of the present disclosure, in the method according to any one of the first to fifteenth aspects, the clone may be a mammalian-derived cell.

According to an eighteenth aspect of the present disclosure, in the method according to any one of the first to fifteenth aspects, the clone may be a CHO cell or a human embryonic kidney cell (HEK cell).

According to a nineteenth aspect of the present disclosure, there is provided an information processing apparatus comprising: one or more processors; and one or more storage devices that store a command to be executed by the one or more processors, in which the one or more processors acquire culture data of one or more types of clones for a clone that produces a useful substance, analyze the culture data and limit the clones to a prediction target, and use data measured for a clone as the prediction target to predict production stability of the useful substance by the clone as the prediction target.

For the information processing apparatus according to the nineteenth aspect, a configuration can be employed in which the same aspect as the method of any one of the second to eighteenth aspects is included.

According to a twentieth aspect of the present disclosure, there is provided a program for causing a computer to implement: a function of acquiring culture data of one or more types of clones for a clone that produces a useful substance; a function of analyzing the culture data and limiting the clones to a prediction target; a function of using data measured for a clone as the prediction target to predict production stability of the useful substance by the clone as the prediction target.

For the program according to the twentieth aspect, a configuration can be employed in which the same aspect as the method of any one of the second to eighteenth aspects is included.

According to a twenty-first aspect of the present disclosure, there is provided a prediction model generation method of generating a prediction model that causes a computer to implement a function of predicting production stability of a clone that produces a useful substance, the prediction model generation method comprising: causing a system including one or more processors to execute: acquiring culture data of one or more types of clones; analyzing the culture data and limiting the clones to a prediction target; performing machine learning using a plurality of pieces of training data in which data measured for a clone corresponding to the prediction target and a ground truth stability label are associated with each other, and training the prediction model such that an output of the prediction model in response to an input of the data approaches the ground truth stability label.

For the prediction model generation method according to the twenty-first aspect, a configuration can be employed in which the same aspect as the method of any one of the second to eighteenth aspects is included.

According to the present disclosure, the prediction target is appropriately limited based on information obtained by analyzing the culture data, so that it is possible to predict the production stability of a clone that produces a useful substance with high accuracy. In addition, according to the present disclosure, the prediction target is limited, so that it is possible to reduce a cost of predicting the production stability and to perform the prediction at a low cost.

### BRIEF DESCRIPTION OF THE DRAWINGS

Fig. 1 is an explanatory diagram showing an outline of a production process of an antibody drug.
Fig. 2 is a graph showing an example of a change in an antibody production amount by a clone.
Fig. 3 is an explanatory diagram outlining a role of a stability prediction artificial intelligence (AI) implemented by the present embodiment.
Fig. 4 is a conceptual diagram of a machine learning model that predicts production stability based on gene expression data.
Fig. 5 is an explanatory diagram showing an outline of a method of predicting the production stability of the clone according to the present embodiment.
Figs. 6A and 6B are tables showing examples of a dataset used for model training and evaluation.
Fig. 7 is a graph showing an example of narrowing down a target based on a certain index of culture data.
Fig. 8 is a table showing the number of clones of five-type antibody-producing CHO cells prepared as evaluation samples and examples of assignment of stability labels.
Fig. 9 is a table showing the number of clones corresponding to the top 40% of relative rankings in values of an antibody production amount for each antibody type and examples of the assignment of the stability labels.
Fig. 10 is a table showing the number of clones corresponding to the top 60% of the relative rankings in values of an integral viable cell density for each antibody type and examples of the assignment of the stability labels.
Fig. 11 is a table showing the number of clones corresponding to the top 40% of the relative rankings in values of lactic acid concentration for each antibody type and examples of the assignment of the stability labels.
Fig. 12 is a block diagram showing a functional configuration of an information processing apparatus according to the embodiment.
Fig. 13 is a block diagram showing an example of a hardware configuration of the information processing apparatus.
Fig. 14 is a block diagram showing an example of a hardware configuration of a machine learning apparatus that executes machine learning processing for generating a production stability prediction model.
Fig. 15 is a flowchart showing an example of a machine learning method executed by the machine learning apparatus.
Fig. 16 is a flowchart showing an example of an information processing method executed by the information processing apparatus according to the embodiment.

### DESCRIPTION OF THE PREFERRED EMBODIMENTS

Hereinafter, a preferred embodiment of the present invention will be described in detail with reference to the accompanying drawings.

### <<Outline of Production Process of Antibody Drug>>

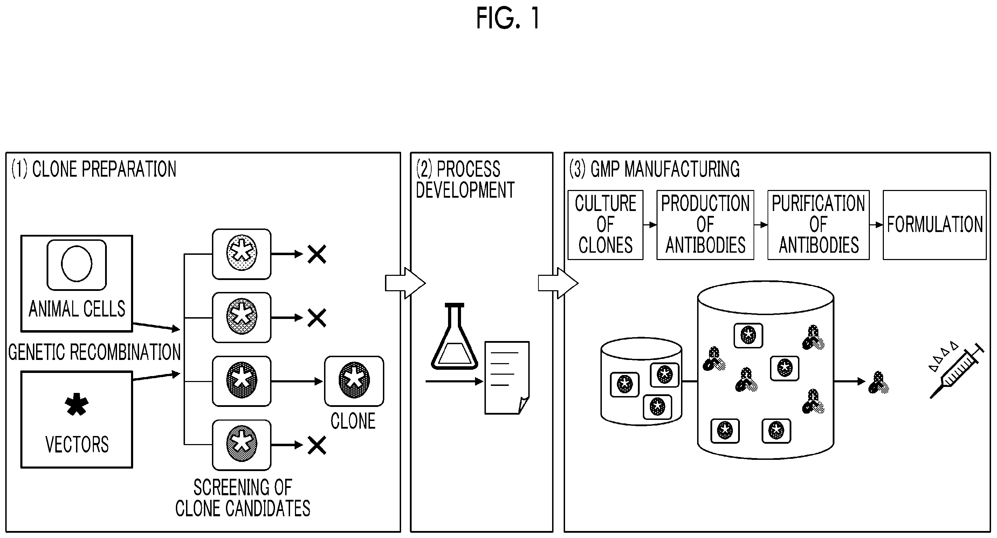

Among biopharmaceuticals, antibody drugs, which are expanding in the market due to their high efficacy and safety, are produced using clones of animal cells capable of stably producing antibodies, which are proteins having complex structures. Hereinafter, an antibody will be described as an example of a useful substance. Fig. 1 is an explanatory diagram showing an outline of a production process of an antibody drug. The process of producing the antibody drug includes [1] a clone preparation phase, [2] a process development phase, and [3] a good manufacturing practice (GMP) manufacturing phase.

The clone preparation phase includes a process of performing genetic recombination on animal cells suitable for antibody drug production by adding vectors to prepare a plurality of clone candidates, and a process of screening the plurality of candidates for clones that excel in terms of an antibody production amount, cell proliferation properties, quality stability which ensures that cell characteristics remain unchanged even after repeated proliferation, and the like.

The process development phase is a phase in which a production process (culture conditions, purification conditions, and the like) required for GMP manufacturing is developed using the clones subjected to the screening.

In the GMP manufacturing phase, under the established production process, the clones are cultured and proliferated, allowing the clones to produce antibodies. Further, by purifying and formulating the antibodies, the antibody drug is completed.

In a case of producing antibodies in clones, it is required that their production capability remains unchanged (stable) over a long period. Therefore, a variety of clones are prepared as extensively as possible, and those with stable production capability are selected from the prepared clones; however, in the related art, experimental verification that requires several months of continuous culture is required, resulting in a high workload.

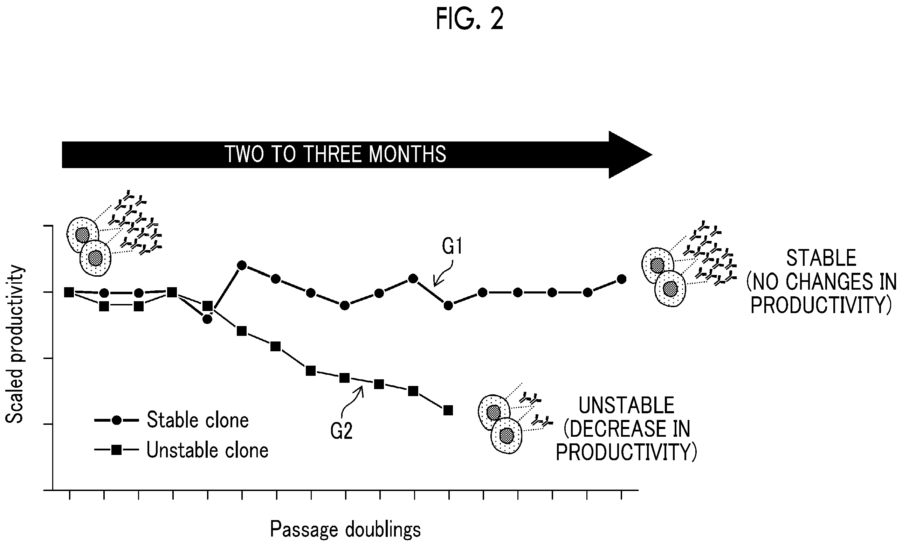

Fig. 2 is a graph showing an example of a change in the antibody production amount by the clone. The vertical axis represents antibody production capability, and the horizontal axis represents elapsed time (a point in time). The "antibody production capability" is represented by the antibody production amount of antibodies produced per unit time by clones.

Fig. 2 shows a graph in which how much the amount of antibodies produced by clones changes over a long period (two to three months) is plotted. A graph G1 is a graph showing a change in the antibody production amount for a clone having stable production capability. A graph G2 is a graph showing a change in the antibody production amount for a clone having unstable production capability. As shown in the graph G1, the clone having stable production capability can maintain production capability that is substantially unchanged from the present point in time, with the production capability that remains substantially unchanged even after two to three months from the present point in time. On the other hand, as shown in the graph G2, the clone having unstable production capability gradually decreases in production capability over two to three months.

In the present invention, the "present point in time" is a point in time at the two-week standard test, or a point in time at which the standard test ends, that is, a point in time at which culture for discriminating the production stability is started. In addition, the "antibody production capability at the present point in time" is the antibody production amount produced per unit time by the clone in the two-week standard test.

In a case in which antibody-producing cells are prepared through gene introduction, both stable clones and unstable clones may be generated, as shown in Fig. 2. Therefore, in the clone preparation phase, a variety of clones are prepared, and a clone having stable production capability, which exhibits behavior like that shown in the graph G1, is selected from among the prepared clones.

The behavior of the production capability as shown in Fig. 2 varies depending on the type of clone, and in the related art, every time the type of antibody to be produced by the clone is changed, the same experiment as that shown in Fig. 2 has to be performed to evaluate the production stability of each clone.

On the other hand, the embodiment of the present disclosure proposes a system for accurately predicting the antibody production stability several months later based on information obtained from the clone at the present point in time. Here, the "information obtained from the clone at the present point in time" is information obtained from the clone in the two-week standard test. The antibody production stability, which is the response variable of the prediction, can be defined as the presence or absence of a change in the antibody production amount between the present point in time and after a culture period of several months. The "several months" here is, for example, a period of two months or more and may be, for example, two to three months. Additionally, the "several months" may be a period until a predetermined number of subcultures are performed. The period settings may be determined based on the proliferation capability of the clone or may be determined based on the culture period of the clone in a case of actually manufacturing the antibody. The "present point in time" is a point in time at the initial culture, which is shown at the left end of the graph in Fig. 2, that is, a point in time at which the two-week standard test ends, and is a point in time at which the culture for discriminating the antibody production stability is started. The production capability being "stable" means that there is no change in the antibody production amount between the present point in time and several months later. "No change" includes a case in which the amount of change is within an allowable range and can be considered to be substantially no change. The production capability being "unstable" means that there is a change in the antibody production amount between the present point in time and several months later, and in many cases, the production amount decreases. A threshold value for considering the change in production capability may be freely set, but may be, for example, ± 30% or ± 20% with respect to the production amount at the present point in time.

### <<Regarding Generalization Performance to Unknown Clones>>

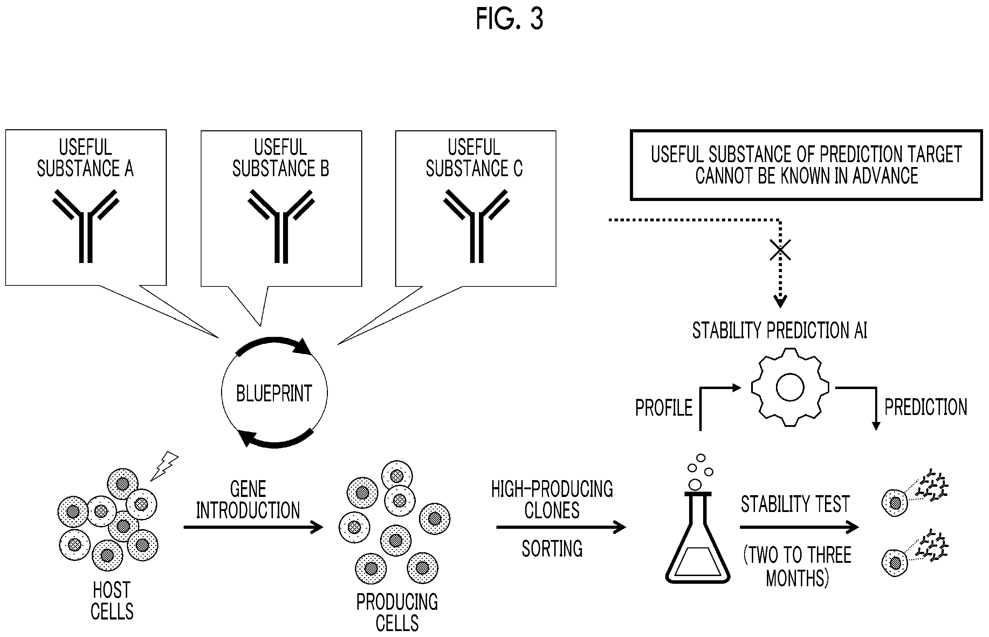

Fig. 3 is an explanatory diagram illustrating a role of a stability prediction artificial intelligence (AI) implemented by the present embodiment. As shown in Fig. 3, in the clone preparation phase, gene introduction is performed to introduce a blueprint of a gene for a desired useful substance into a host cell. For example, in a case in which the blueprint for producing a useful substance A is introduced into the host cell through gene introduction, cells that produce the useful substance A are obtained. Since such producing cells are produced probabilistically, cells that do not produce the useful substance A or cells that have an insufficient production amount may also be generated. Therefore, first, a simple test is performed at this stage, and a high-producing clone capable of sufficiently producing the useful substance A is selected.

Thereafter, in a case of the related art, as described with reference to Fig. 2, a stability test for two to three months is performed to confirm whether the useful substance A can be continuously produced over several months, and a clone having production stability is selected.

In the present embodiment, as an alternative to the stability test in the related art, a stability prediction AI is constructed, and a state (change in production capability) after two to three months is predicted by the stability prediction AI based on a profile obtained by measuring the state of the clone at the present point in time, that is, the state of the clone at the two-week standard test.

Since the types of useful substances (for example, antibodies) to be produced by the cells are diverse depending on the purpose, it is desirable to construct a model that can predict production stability regardless of the type of useful substance produced by the cells. That is, a model that robustly predicts antibody production stability for an unknown antibody type is preferable.

In a case of training a model to be applied to the stability prediction AI, a target useful substance cannot be known in advance, and the type of useful substance used during the model training may be different from the type of useful substance produced by clones for which predictions are made after training. That is, a model that robustly and accurately predicts the production stability for an unknown useful substance type is preferable, and it is preferable to construct a prediction model having domain generalization with the useful substance type as a domain.

### <<Outline of Machine Learning Model That Predicts Production Stability>>

In the present embodiment, in the clone preparation phase, a stability prediction AI that makes it possible to estimate (predict) the presence or absence of a change in production capability two to three months later, that is, predict the production stability of the useful substance, based on the information on the clone at the present point in time, is constructed. More specifically, a model that receives an input of gene expression data of the clone at the present point in time (at the time of the standard test) and that outputs a stability label indicating the production stability of the useful substance is constructed. In more detail, a portion of the clones is used as clones for the standard test, and another portion is used as clones subjected to genetic analysis for acquiring the gene expression data, thereby acquiring the gene expression data of the clones used in the standard test. The stability label can be represented by a binary value in which a value of "1" indicates "stable" and a value of "0" indicates "unstable". The prediction model that predicts the production stability may be a two-class classification model that performs class classification into "stable" or "unstable".

The gene expression data includes one or more gene levels. The gene expression data used in the present embodiment includes data obtained by quantifying the gene expression level of each of a plurality of genes. The gene expression data can be obtained by, for example, ribonucleic acid (RNA) sequence analysis. The value indicating the gene expression amount is, for example, a count value that is a positive integer, and can be logarithmically transformed and used as a feature amount.

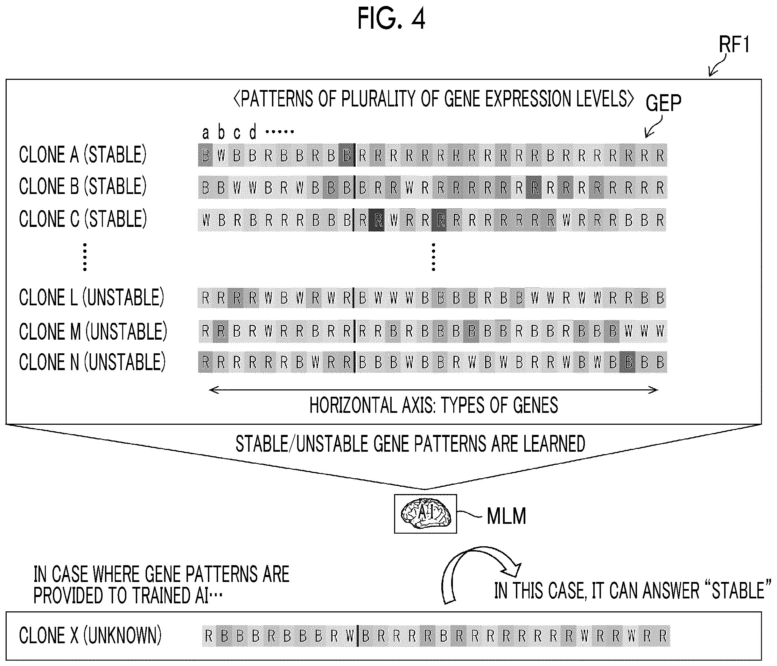

Fig. 4 is a conceptual diagram of a machine learning model MLM that predicts the production stability based on the gene expression data. An example of a dataset of training data is shown inside a rectangular frame RF1 in Fig. 4. In Fig. 4, the gene expression data at the present point in time (at the time of the standard test) of each of a plurality of clones A to N is represented as a gene expression pattern GEP visualized by a heatmap. The horizontal axis of the gene expression pattern GEP represents the type of gene, and the gene expression level of each of a plurality of genes is represented by a two-color gradation (heatmap). The number of types of genes a, b, c, d, ... included in the gene expression data is, for example, preferably 300 to 400 types selected by acquiring all pieces of gene expression data for stable clones and unstable clones and using the statistical significance probabilities between the two groups of the stable clones and the unstable clones. Further, in a case in which the number of types of genes is narrowed down, it is preferable to actually train the machine learning model MLM while increasing and decreasing the number of types of genes using the selected genes, to search for the number of types of genes having high prediction performance, and to narrow down the number of types of genes to, for example, 50 to 100 types of genes. Here, although all the pieces of gene expression data are acquired, it is not always necessary to acquire all the pieces of gene expression data, and some of genes may be randomly selected, and the gene expression data of the selected genes may be acquired. Due to the limitations of the illustration, the colors of the heatmap cannot be represented; instead, red is denoted as "R," blue is denoted as "B," and white is denoted as "W". Red (R) represents that the gene expression level is relatively high, and blue (B) represents that the gene expression level is relatively low. White (W) represents that the gene expression level is an intermediate value.

Each of the plurality of clones A to N has been confirmed to be either "stable" or "unstable" based on experimental verification from several months of culture following the standard test, and the stability label (ground truth label) indicating "stable" or "unstable" is assigned to each of the clones A to N. In this way, a dataset including a plurality of pieces of training data in which the gene expression data at the present point in time for each of the plurality of clones A to N and the ground truth stability label are associated with (linked to) each other is prepared. Then, the machine learning model MLM is trained using the plurality of pieces of training data, allowing the machine learning model MLM to learn the stable or unstable gene pattern. In a case in which the gene expression data of an unknown clone X at the present point in time (at the time of the standard test) is input to the trained machine learning model MLM that has been trained in this way, the machine learning model MLM predicts the production stability based on the input gene expression data and outputs a "stable" or "unstable" label as a prediction result. In Fig. 4, an example is shown in which the machine learning model MLM predicts that an unknown clone X is "stable".

### <<Outline of Embodiment: Constructing Model That Predicts Production Stability of Useful Substance by Limiting Prediction Target>>

Since the clones that produce useful substances have various characteristics, it is difficult to predict the production stability of all the clones with high accuracy regardless of the type. In the present embodiment, by limiting the prediction target based on an index obtained from culture data of each clone at the present point in time (at the time of the standard test), highly accurate predictions are achieved. Here, the culture data is general data that can be measured using a culture device or a dedicated device by partially sampling a culture liquid containing cells for the clone.

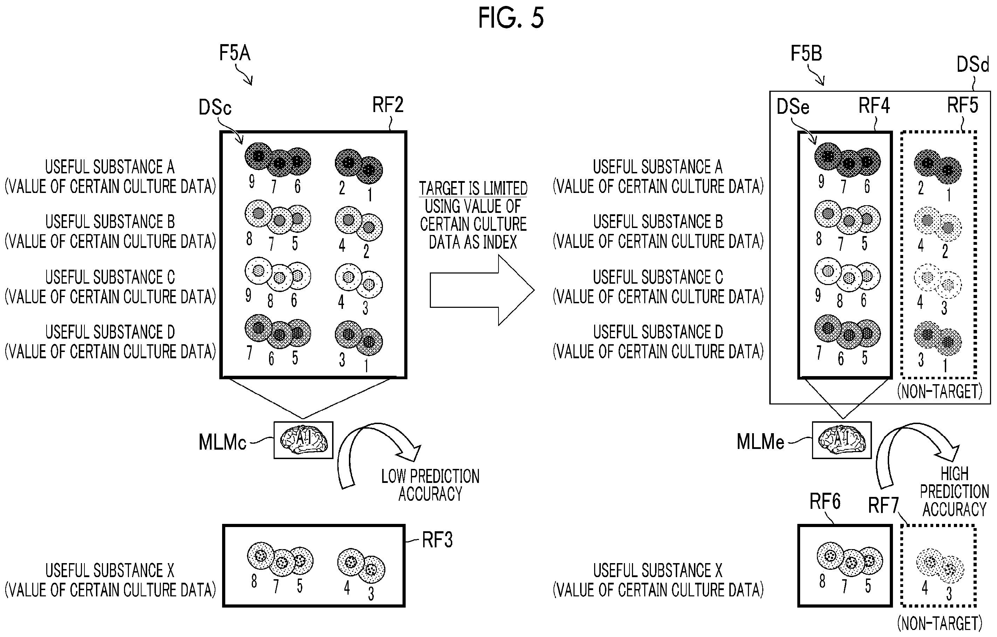

Fig. 5 is an explanatory diagram showing an outline of a method of predicting the production stability of the useful substance from the clone according to the present embodiment. A left diagram F5A of Fig. 5 shows a comparative example in a case in which the prediction target is not limited, and a right diagram F5B of Fig. 5 shows an outline of a method according to the present embodiment.

A case in which the prediction target is not limited, as shown in the left diagram F5A, will be described. A dataset DSc including training data of a plurality of types of clones that produce useful substances A to D is schematically shown in a rectangular frame RF2 in the left diagram F5A. This dataset DSc includes training data for a total of 20 clones, with five clones for each of the useful substances A to D. Here, the training data is data in which the gene expression data at the time of the standard test and the ground truth stability label are associated with (linked to) each other for each of 20 clones. The value displayed below each clone in Fig. 5, such as "9", "7", and "6", represents the measured value of certain culture data for each clone at the time of the standard test. The value may represent a relative level for each clone, which can be acquired from the measured value, rather than the measured value. Here, the left diagram F5A has been described, but the same applies to the right diagram F5B.

The left diagram F5A shows that a machine learning model MLMc is trained using all the pieces of training data of the dataset DSc without limiting the training data and that the production stability of a plurality of types of clones that produce an unknown useful substance X is predicted using the trained model. In this case, without particularly limiting the plurality of types of clones that produce the unknown useful substance X, which are prediction targets, the prediction of the production stability is performed for all five types of clones that produce the unknown useful substance X as shown in a rectangular frame RF3. The prediction of the production stability is performed by acquiring the gene expression data at the present point in time (at the time of the standard test) for all the five types of clones that produce the unknown useful substance X and inputting the gene expression data into the trained model, but the prediction accuracy is low.

Next, the method according to the present embodiment shown in the right diagram F5B will be described. In the method shown in the right diagram F5B, the prediction target is limited using a value of certain culture data at the time of the standard test as an index, as compared to the method in which the prediction target is not limited as shown in the left diagram F5A. First, a threshold value is determined by focusing on the value of certain culture data, and a population of clones included in a dataset DSd is grouped. For example, the population of clones is divided into two groups: one group having a value of the culture data, which is used as the index, that is relatively greater than the threshold value, and another group having a smaller value. Here, an example is shown in which the threshold value is set to "5", a population having a value of the culture data of "5" or greater, which is used as the index, is set as a target for training, and a population having a value of the culture data of less than "5" is excluded from the target. Through this threshold value processing, as shown in a rectangular frame RF4, training data of a total of 12 clones, with three clones for each of the useful substances A to D, is retained as the target, and a dataset DSe including the training data of this limited population is used to train a machine learning model MLMe. On the other hand, training data of eight clones shown in a dashed rectangular frame RF5, that is, training data of clones that do not satisfy the condition of the threshold value, is excluded from the processing target.

In this way, the machine learning model MLMe is trained using the dataset DSe in which the target is limited. In a case of predicting the production stability of the clones that produce the unknown useful substance X by using the trained model, the clones as the prediction targets are limited to those satisfying the limitation condition by the threshold value (a population having a value of the index greater than the threshold value) by applying the threshold value to the value of the culture data, which is used as the index, thereby performing the prediction, as in the population of the clones of the dataset DSe used for model training. Three types of clones shown in a rectangular frame RF6 represent clones corresponding to the prediction targets. In addition, two types of clones shown in a dashed rectangular frame RF7 represent clones that are excluded from the prediction targets. By limiting the prediction target in this manner and performing the prediction, high prediction accuracy can be achieved. Further, since the clones shown in the dashed rectangular frame RF7, which are excluded from the prediction targets, do not require the acquisition of the gene expression data, the cost of genetic analysis can be reduced.

### <<Example of Dataset Used for Training and Evaluation>>

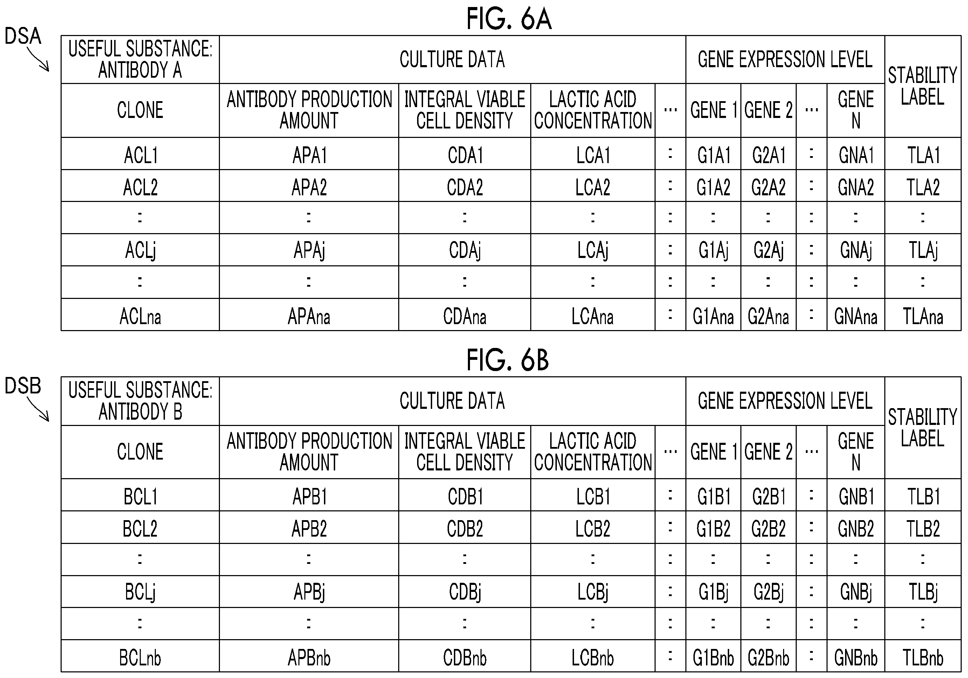

Figs. 6A and 6B show examples of a dataset used for model training and evaluation. Fig. 6A shows an example of a dataset DSA for a clone that produces an antibody A as a useful substance, and Fig. 6B shows an example of a dataset DSB for a clone that produces an antibody B as a useful substance. Although not shown, the same applies to a dataset for a clone that produces another type of antibody as a useful substance.

The dataset DSAincludes culture data at the time of the standard test, which is measured for each of a plurality clones ACLj, gene expression data at the time of the standard test, and a ground truth stability label obtained through the stability test. A subscript j represents an index number for identifying the clone. The culture data may include, for example, one or more items such as an antibody production amount, an integral viable cell density (IVCD), a lactic acid concentration, and a pH. The culture data may be general data that can be measured using the culture device or the dedicated device by partially sampling a culture liquid containing cells, and may include, for example, one or more of the total number of cells, the amount of cell secreted substances, the amount of cell-produced substances, the amount of cell metabolic substances, and the amount of medium components. A character symbol in each cell of the table shown in Fig. 6A (a symbol with the subscript j) represents a value of the corresponding data item.

The same applies to the dataset DSB. The number na of the clones ACLj included in the dataset DSA and the number nb of the clones BCLj included in the dataset DSB may be different from each other.

In this manner, by focusing on the index of certain culture data from prepared datasets of a plurality of domains (useful substance types), the target is narrowed down (limited).

### <<Example of Narrowing Down Prediction Target>>

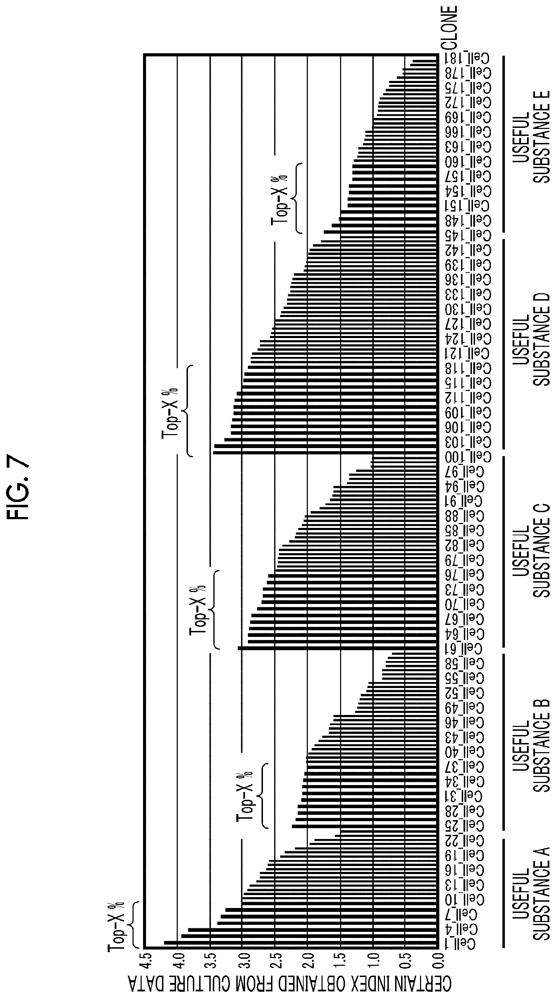

Fig. 7 is a graph showing an example of narrowing down the prediction target based on a certain index of culture data. A plurality of types of clones that produce each of a plurality of useful substances A to E are arranged on the horizontal axis. The vertical axis is a value of a certain index obtained from the culture data at the time of the standard test. The clone shown in Fig. 7 is a clone used for model training (learning).

As shown in Fig. 7, the distribution ranges of the certain index obtained from the culture data may differ depending on clones that produce different useful substance types. In this case, as described with reference to Fig. 5, in a case in which the threshold value is determined for the value of the index, the clones are divided into two populations based on a relative magnitude relationship with the threshold value, and the population of the clones to be used for training and the population of the clones to be excluded from training are determined, variations occur in the number of clones to be subjected to the training depending on the useful substance type to be produced. For example, in a case in which the threshold value of the index is set to 2.5 and a population of clones having a value equal to or greater than the threshold value is used for training, clones that produce a useful substance B are not used for training.

Therefore, for example, as shown in Fig. 7, the training target may be limited to a relative top X% of clones that produce each of the useful substances A to D based on a certain index obtained from the culture data. Here, the relative top X% means the top X% in a case in which, in the population of the clones that produce each of the useful substances A to D, clones are arranged in descending order based on a certain index obtained from the culture data. It is preferable that the criterion "X%" corresponding to the threshold value as the limitation condition is adjusted such that the number of samplings from each of the useful substances A to E is substantially the same. The relative top X% is an example of a "threshold value defined using a ranking of the value of the index" in the present disclosure.

Even in a case in which the training target is limited in this manner, there may be a clone having stable production capability and a clone having unstable production capability. Then, even in a case of predicting the production stability of a clone that produces an unknown useful substance Y using the trained model, the clones as the prediction targets are limited to the top X% of clones based on a certain index obtained from the culture data, as in the clones used for model training.

Here, a plurality of types of clones that produce each of the plurality of useful substances A to E are used for model training, but it is not always necessary to use a plurality of types of clones that produce different useful substances, and for example, only a clone that produces the useful substance A may be used for training. In this case, as the method of limiting the population of clones used for training, by focusing on the value of certain culture data at the time of the standard test and setting the threshold value, the population may be limited based on the relative magnitude relationship with the threshold value, or the top X% based on the value of a certain index obtained from the culture data at the time of the standard test may be used. Additionally, although the relative top X% is used, a relative bottom X% may be used depending on a certain index obtained from the culture data.

The index of the culture data and the threshold value in a case of limiting the prediction target may be determined through a process of trial and error, repeatedly performing hypothesis and verification, based on the prepared dataset. Alternatively, the index of the culture data and the threshold value in a case of limiting the prediction target can be determined by performing exploratory analysis based on the prepared dataset.

For example, in a case in which there are datasets of five useful substances A to E (domains) as shown in Fig. 7, an information processing apparatus including a processor uses a feature selection method such as a filter method to evaluate a degree of association between each feature amount and the response variable (stability label) in each of the five domains and sets the feature amount having a high degree of association in, for example, four or more domains among the five domains as a feature amount having high domain universality. The information processing apparatus extracts data that satisfies a specific condition as a subset by focusing on a certain index from among all pieces of data and performs domain generalization evaluation for the extracted subset based on the number of feature amounts having high domain universality. In a case in which the number of feature amounts having high domain universality is large, it is evaluated as a subset having high domain generalization. By training the prediction model using the data of the subset having high domain generalization as the training data, the trained model can robustly predict the production stability even for other domains (useful substance types) for a population (subset) in which the target is limited under the same conditions as during training.

In a case of antibody-producing clones, effective indices of the culture data for limiting the prediction target are, for example, the antibody production amount, the integral viable cell density, the lactic acid concentration, and the like, and it has been confirmed that targeting the top population of the values of any one of these indices enables highly accurate production stability prediction.

### <<Examples of Useful Substances>>

The useful substance is not limited to an antibody and may be an antibody-like protein. The useful substance may be any of a protein, a peptide, or a virus, which is a pharmaceutical raw material.

### <<Example of Clone>>

The clone that produces a useful substance may be a vertebrate-derived cell. The clone may be, for example, a mammalian-derived cell. The clone may be a CHO cell or a HEK cell.

### <<Examples>>

Hereinafter, Examples 1 to 3 to which the technology of the present disclosure is applied will be described. The common configurations among Examples 1 to 3 are as follows. That is, the useful substance is an antibody, and the producing cell is a CHO cell. An example is shown in which, for clones of five-type antibody-producing CHO cells as evaluation samples, a plurality of types of clones are prepared for each type, 100 types of gene expression levels are selected from the total gene expression level measured in the two-week standard test using RNA sequence (RNA-Seq) analysis as explanatory variables, a logistic regression model that classifies the samples into two classes, that is, stable or unstable, is used as a learning device, and a five-fold cross-validation is carried out to train a prediction model, and the performance evaluation is performed using the area under the precision-recall curve (PRAUC). The number of types of gene expression levels used as explanatory variables was set to 100 by actually training the prediction model while increasing and decreasing the number of types using 300 to 400 types of genes selected using statistical significance probabilities, and searching for the number of types with high prediction performance, in Examples 1 to 3. In the standard test, the number of clones (CHO cells) seeded was 5 × 10^5 cells/mL, and suspension culture was performed in a 40 mL flask.

In the five-fold cross-validation, the samples were divided into five antibody types, and the performance was evaluated based on an antibody type that was not used for training. That is, datasets of four antibody types were used as data for training (learning), and a dataset of the remaining one antibody type was used as test data for performance evaluation.

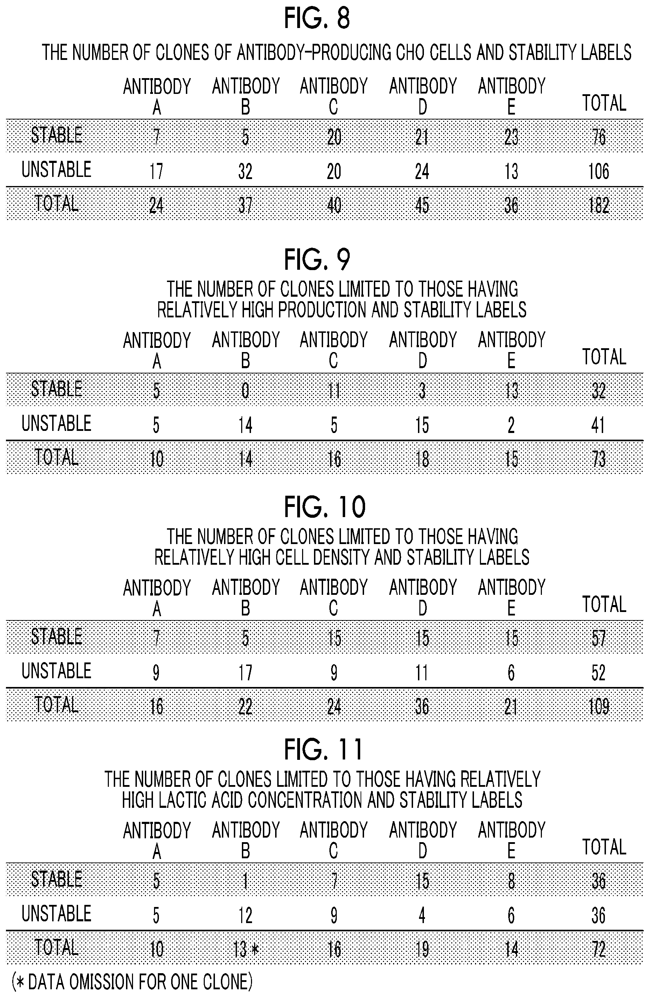

Fig. 8 is a table showing the number of clones of five-type antibody-producing CHO cells prepared as evaluation samples and examples of assignment of the stability labels. As evaluation samples, 182 clones of five-type antibody-producing cells were prepared, and the cells were cultured for 2 months under the same conditions as in the standard test, so that the stability label ("stable" or "unstable") is assigned to each clone (refer to Fig. 8). For example, there are a total of 24 clones that produce the antibody A, of which 7 clones are labeled as "stable" and 17 clones are labeled as "unstable". In addition, for each of the 182 clones, culture data and gene expression data are acquired at the time of the standard test, and the gene expression data and the stability label are linked to each other for each clone, thereby forming the training data.

### [Example 1]

In Example 1, an example of performing the stability prediction in which the prediction target is limited to a "relatively high-producing clone" will be described. Here, the "relatively high-producing clone" means a clone having a relatively high production amount of the useful substance.

A method of limiting clones to the training target to be used to train the prediction model in a case of performing the stability prediction in which the prediction target is limited to the "relatively high-producing clone" will be described. Limiting the clones to the training target corresponds to limiting the clones to the target be predicted by the prediction model in the training, that is, limiting the clones to the prediction target by the prediction model.

As the method of limiting clones to the training target, a method of focusing on the "antibody production amount" based on the culture data at the time of the standard test of all the 182 clones, searching for the threshold value such that the prediction performance of the prediction model is high, and limiting the clones to the top 40% of the relative rankings in each antibody type was used. Here, the "antibody production amount" can be, for example, a cumulative amount of antibody production over a period of two weeks (14 days) in the standard test. Alternatively, the antibody production amount may be a cumulative amount of antibody production over a certain period during the standard test, for example, a period, for example, a period of 10 days, or may be an antibody production amount per unit time obtained by dividing the cumulative amount by a measurement period. The "top 40%" is an example of the threshold value. Fig. 9 is a table showing the number of clones corresponding to the top 40% of the relative rankings in values of the antibody production amount for each antibody type and examples of the assignment of the stability labels.

Fig. 9 shows an example of a total of 73 clones corresponding to the top 40% of the relative rankings for each antibody type. The five-fold cross-validation was carried out using a dataset for each antibody type, which includes training data in which the gene expression data at the time of the standard test of the 73 clones shown in Fig. 9 and the stability labels are linked to each other. As a result of limiting the training target in this manner, the prediction performance of the trained prediction model was a PRAUC value of 0.743. For the clones as the prediction targets in a case of predicting the production stability of an unknown useful substance by using the trained prediction model, the prediction is performed by analyzing the culture data at the time of the standard test (at the present point in time) and limiting the clones to the top 40% of the "antibody production amount", in the same manner as in limiting the training target.

### [Comparative Example]

In contrast, the prediction performance of the prediction model according to a comparative example obtained in a case in which the same training was performed using a dataset including all pieces of data of the 182 clones shown in Fig. 8 without limiting the training target and the five-fold cross-validation was carried out was a PRAUC value of 0.503. The prediction target is not limited in the same manner as the training target. The performance of the prediction model with the prediction target limited according to Example 1 was confirmed to be more accurate than that of the prediction model according to the comparative example.

This result shows that the prediction model generated using the method of Example 1 can accurately predict the production stability for unknown useful substances. At the same time, it is considered that limiting the clones to the relatively high-producing clones enables limiting the clones to targets that can be predicted with high accuracy without any hindrance to the selection process for producing clones of the useful substances, which leads to reduced costs and makes the stability prediction according to the present disclosure feasible.

### [Example 2]

In Example 2, an example of performing the stability prediction in which the prediction target is limited to a "clone having a relatively high cell density" will be described. First, a method of limiting clones to the training target for training the prediction model in a case of performing the stability prediction in which the prediction target is limited to the "clone having a relatively high cell density" will be described. In the same manner as in Example 1, a method of focusing on the "integral viable cell density (IVCD)" based on the culture data at the time of the standard test of all the 182 clones shown in Fig. 8, searching for the threshold value such that the prediction performance of the prediction model is high, and limiting the clones to the top 60% of the relative rankings in each antibody type was used. Here, the "clone having a relatively high cell density" can be acquired, for example, based on the "integral viable cell density (IVCD)" over a period of two weeks (14 days) in the standard test. Alternatively, the "clone having a relatively high cell density" may be acquired based on the "integral viable cell density (IVCD)" over a certain period during the standard test, for example, over a period of 10 days. The "top 60%" is an example of the threshold value. Fig. 10 is a table showing the number of clones corresponding to the top 60% of the relative rankings in values of the integral viable cell density for each antibody type and examples of the assignment of the stability labels.

Fig. 10 shows an example of a total of 109 clones corresponding to the top 60% of the relative rankings for each antibody type. The five-fold cross-validation was carried out using a dataset for each antibody type, which includes training data in which the gene expression data at the time of the standard test of the 109 clones shown in Fig. 10 and the stability labels are linked to each other. As a result of limiting the training target in this manner, the prediction performance of the trained prediction model was a PRAUC value of 0.647. That is, the performance of the prediction model with the prediction target limited according to Example 2 was confirmed to be more accurate than the PRAUC (0.503) of the prediction model according to the comparative example in which the target is not limited. For the clones as the prediction targets in a case of predicting the production stability of an unknown useful substance by using the trained prediction model, the prediction is performed by analyzing the culture data at the time of the standard test (at the present point in time) and limiting the clones to the top 60% of the "integral viable cell density (IVCD), in the same manner as in limiting the training target.

This result shows that the prediction model generated using the method of Example 2 can accurately predict the production stability for unknown useful substances. At the same time, limiting the clones to those with a relatively high viable cell density enables limiting the clones to targets that can be predicted with high accuracy without hindering the selection process for producing clones of the useful substances, which leads to reduced costs and makes the stability prediction according to the present disclosure feasible.

### [Example 3]

In Example 3, an example of performing the stability prediction in which the prediction target is limited to a "clone having a relatively high lactic acid concentration" will be described. First, a method of limiting clones to the training target for training the prediction model in a case of performing the stability prediction in which the prediction target is limited to the "clone having a relatively high lactic acid concentration" will be described. In the same manner as in Example 1, the "lactic acid concentration" of each clone is acquired using the median value of the "lactic acid concentration" of the culture liquid measured at each point in time, for example, every day, within two weeks (14 days) as a representative value by focusing on the "lactic acid concentration" of the culture liquid in which clones are cultured, based on the culture data of the two-week standard test for all the 182 clones shown in Fig. 8. Then, a method of searching for the threshold value such that the prediction performance of the prediction model is high and limiting the clones to the top 40% of the relative rankings in each antibody type was used. The "top 40%" is an example of the threshold value. Fig. 11 is a table showing the number of clones corresponding to the top 40% of the relative rankings in values of the lactic acid concentration for each antibody type and examples of the assignment of the stability labels.

Fig. 11 shows an example of a total of 72 clones corresponding to the top 40% of the relative rankings for each antibody type. The reason there is one fewer clone as compared to Fig. 9 is due to a data omission for one clone in the measurement of the lactic acid concentration.

The five-fold cross-validation was carried out using a dataset for each antibody type, which includes training data in which the gene expression data at the time of the standard test of the 72 clones shown in Fig. 11 and the stability labels are linked to each other. As a result of limiting the target in this manner, the prediction performance of the trained prediction model was a PRAUC value of 0.613. That is, the performance of the prediction model with the prediction target limited according to Example 3 was confirmed to be more accurate than the PRAUC (0.503) of the prediction model according to the comparative example in which the target is not limited. For the clones as the prediction targets in a case of predicting the production stability of an unknown useful substance by using the trained prediction model, the prediction is performed by analyzing the culture data at the time of the standard test (at the present point in time) and limiting the clones to the top 40% of the "lactic acid concentration", in the same manner as in limiting the training target.

This result shows that the prediction model can accurately predict the production stability for unknown useful substances. At the same time, limiting the clones to those with a relatively high lactic acid concentration enables limiting the clones to targets that can be predicted with high accuracy without hindering the selection process for producing clones of the useful substances, which leads to reduced costs and makes the stability prediction according to the present disclosure feasible.

### <<Configuration Example of Information Processing Apparatus>>

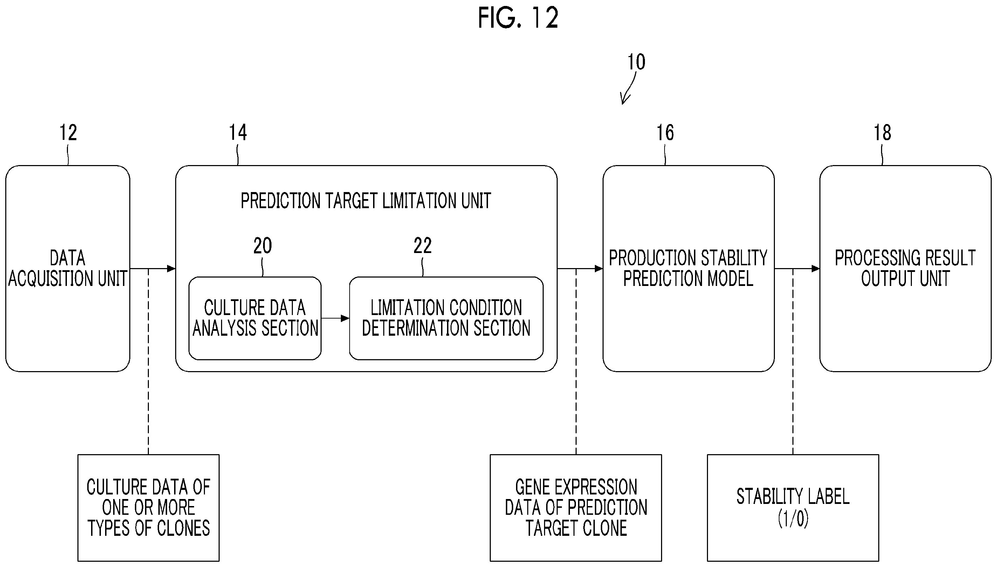

Fig. 12 is a block diagram showing a functional configuration of an information processing apparatus 10 according to the embodiment. The information processing apparatus 10 comprises a data acquisition unit 12, a prediction target limitation unit 14, a production stability prediction model 16, and a processing result output unit 18. Various functions of the information processing apparatus 10 can be implemented by a combination of hardware and software of a computer. The physical form of the information processing apparatus 10 is not particularly limited and may be a server computer, a workstation, a personal computer, a tablet terminal, or the like.

The data acquisition unit 12 acquires various kinds of data including the culture data and the gene expression data of one or more types of clones for a clone that produces a useful substance.

The prediction target limitation unit 14 includes a culture data analysis section 20 and a limitation condition determination section 22 and analyzes the input culture data of one or more types of clones and limits the clones to the prediction target. The culture data analysis section 20 analyzes the culture data. The limitation condition determination section 22 limits the target using the threshold value based on the analysis result of the culture data. For convenience of description, the culture data analysis section 20 and the limitation condition determination section 22 are described separately, but the limitation condition determination section 22 may be included in the culture data analysis section 20. Additionally, the culture data analysis section 20 may be understood to function as the prediction target limitation unit 14.

The culture data analysis section 20 can execute processing of determining the index and the threshold value for limiting the prediction target based on the input dataset. The index and the threshold value that are the limitation conditions for the prediction target may be set based on the analysis result by the culture data analysis section 20 or may be set by the prediction target limitation unit 14 as known information that is grasped in advance by the result of search processing or the like using another information processing apparatus (not shown) or the like.

A machine learning model is applied to the production stability prediction model 16. The production stability prediction model 16 may be a two-class classification model that receives an input of the gene expression data of the clone as the prediction target at the present point in time and that predicts the production stability of the clone based on the input gene expression data to output the stability label. The production stability prediction model 16 is trained using the training data in which the target is limited through the method described in the right diagram F5B of Fig. 5. The gene expression data input to the production stability prediction model 16 includes one or more gene expression levels. The gene expression data input to the production stability prediction model 16 may include data on the expression levels of a plurality of genes. The feature amount used as the explanatory variable may be selected using a known feature amount selection method.

The processing result output unit 18 outputs a processing result including the prediction result of the production stability prediction model 16. The processing result output unit 18 may be configured to perform, for example, at least one of processing of displaying the processing result, processing of recording the processing result in a database or the like, or processing of printing the processing result.

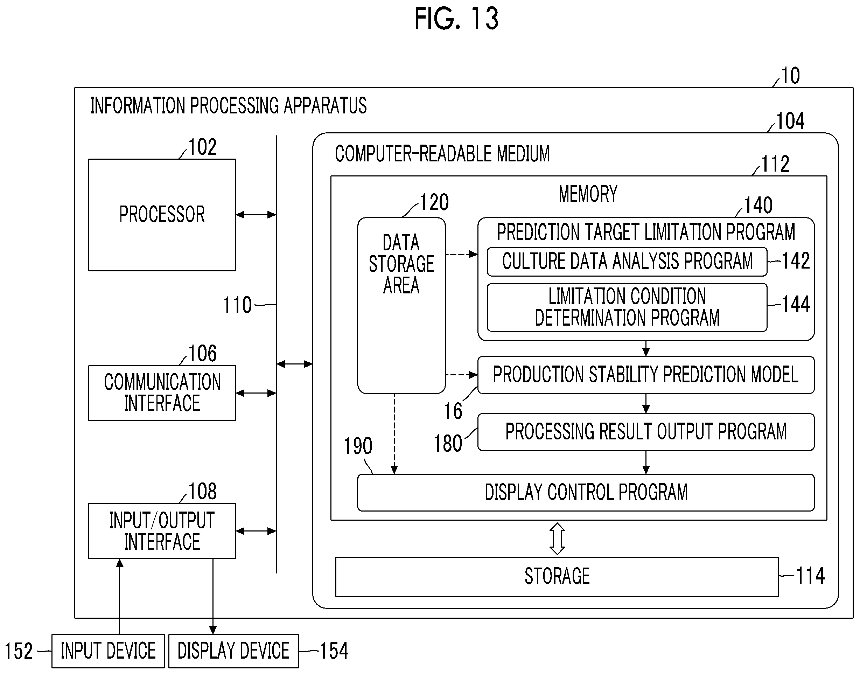

Fig. 13 is a block diagram showing an example of a hardware configuration of the information processing apparatus 10. Although an example of implementing a processing function of the information processing apparatus 10 by using a single computer is described here, the processing function of the information processing apparatus 10 may be implemented by a computer system configured using a plurality of computers.

The information processing apparatus 10 comprises a processor 102, a non-transitory tangible computer-readable medium 104, a communication interface 106, an input/output interface 108, and a bus 110. The processor 102 is connected to the computer-readable medium 104, the communication interface 106, and the input/output interface 108 via the bus 110.

The processor 102 includes a central processing unit (CPU). The processor 102 may include a graphics processing unit (GPU). The computer-readable medium 104 includes a memory 112, which is a main storage device, and a storage 114, which is an auxiliary storage device. For example, the computer-readable medium 104 may be a semiconductor memory, a hard disk drive (HDD) device, a solid state drive (SSD) device, or a plurality of combinations of these. The computer-readable medium 104 is an example of a "storage device" in the present disclosure.

The computer-readable medium 104 includes a data storage area 120 that stores various kinds of data such as the culture data and the gene expression data of one or more types of clones. In addition, the computer-readable medium 104 stores a plurality of programs including a prediction target limitation program 140, the production stability prediction model 16, a processing result output program 180, and a display control program 190, data, and the like. The term "program" includes the concept of a program module and includes a command equivalent to the program. The processor 102 functions as various processing units by executing the commands of the programs stored in the computer-readable medium 104.

The prediction target limitation program 140 includes a command to execute processing of analyzing the culture data and limiting the prediction target. The prediction target limitation program 140 may include a culture data analysis program 142 and a limitation condition determination program 144. The culture data analysis program 142 includes a command to execute processing of analyzing the culture data of one or more types of clones. The culture data analysis program 142 may include a command to execute processing of searching for the index and the threshold value for narrowing down the prediction target based on the dataset.

The limitation condition determination program 144 includes a command to execute processing of limiting the prediction target based on the index and the threshold value determined as the limitation conditions by using the analysis result of the culture data analysis program 142.

The production stability prediction model 16 includes a command to execute processing of predicting the production stability by receiving an input of the gene expression data of the clone related to the prediction target that satisfies the limitation condition.

The processing result output program 180 includes a command to execute processing of outputting the processing result including the production stability predicted by the production stability prediction model 16. The display control program 190 includes a command to execute display control of a display device 154 by generating a display signal necessary for the display output to the display device 154.

The communication interface 106 performs communication processing with an external device by wire or wirelessly and exchanges information with the external device. The information processing apparatus 10 is connected to a communication line (not shown) via the communication interface 106. The communication line may be a local area network, a wide area network, or a combination of these. The communication interface 106 can function as the data acquisition unit that receives an input of data.

The information processing apparatus 10 may comprise an input device 152 and the display device 154. The input device 152 is configured with, for example, a keyboard, a mouse, a multi-touch panel, another pointing device, a voice input device, or an appropriate combination of these. For example, the display device 154 is configured with, for example, a liquid crystal display, an organic electro-luminescence (OEL) display, a projector, or an appropriate combination of these. The input device 152 and the display device 154 are connected to the processor 102 via the input/output interface 108. The input device 152 and the display device 154 may be integrally configured as in the touch panel, or the information processing apparatus 10, the input device 152, and the display device 154 may be integrally configured as in the touch panel type tablet terminal.

### <<Configuration Example of Machine Learning Apparatus>>

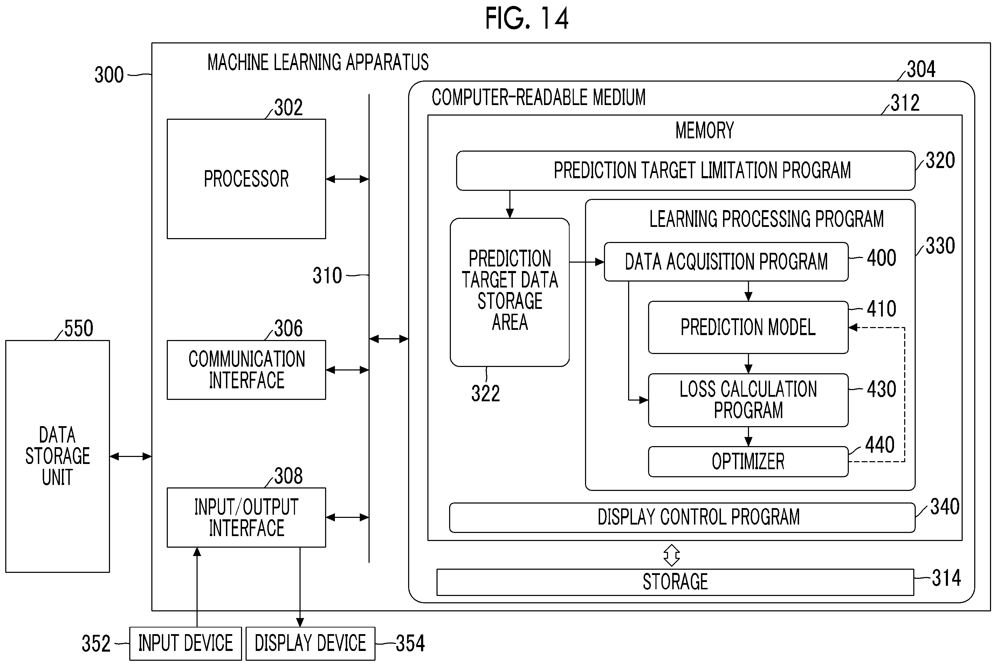

Fig. 14 is a block diagram showing an example of a hardware configuration of a machine learning apparatus 300 that executes machine learning processing for generating the production stability prediction model 16. Here, an example of implementing the processing function of the machine learning apparatus 300 by using a single computer is described, but the processing function of the machine learning apparatus 300 may be implemented by a computer system configured using a plurality of computers.

The machine learning apparatus 300 comprises a processor 302, a non-transitory tangible computer-readable medium 304, a communication interface 306, an input/output interface 308, and a bus 310. The computer-readable medium 304 includes a memory 312 and a storage 314. The processor 302 is connected to the computer-readable medium 304, the communication interface 306, and the input/output interface 308 via the bus 310. An input device 352 and a display device 354 are connected to the bus 310 via the input/output interface 308.

The hardware configuration of the machine learning apparatus 300 may be the same as the corresponding elements of the information processing apparatus 10 described with reference to Fig. 6. The form of the machine learning apparatus 300 may be a server computer, a personal computer, or a workstation. The machine learning apparatus 300 is an example of a "system including one or more processors" in the present disclosure.

The machine learning apparatus 300 is connected to a communication line (not shown) via the communication interface 306 and is communicably connected to an external device such as a data storage unit 550. The data storage unit 550 includes a storage in which a dataset including a plurality of pieces of training data is stored. The data storage unit 550 may store a dataset including all pieces of data of a plurality of domains as illustrated in Figs. 6A and 6B or may store a dataset including data of only a sample of a target limited as the prediction target. The data storage unit 550 may be constructed in the storage 314 in the machine learning apparatus 300.

The computer-readable medium 304 stores a plurality of programs including a prediction target limitation program 320, a learning processing program 330, and a display control program 340, data, and the like. The prediction target limitation program 320 may be the same as the prediction target limitation program 140 described with reference to Fig. 12. The display control program 340 may be the same as the display control program 190 described with reference to Fig. 12.

The computer-readable medium 304 includes a prediction target data storage area 322. The prediction target data storage area 322 stores the training data corresponding to the limited prediction target. The corresponding training data may be sampled as needed by the prediction target limitation program 320 from the dataset stored in the data storage unit 550, or a dataset of only the prediction target may be extracted in advance as a subset.

The learning processing program 330 includes a data acquisition program 400, a prediction model 410 which is a machine learning model, a loss calculation program 430, and an optimizer 440. The data acquisition program 400 includes a command to execute processing of acquiring the training data from the prediction target data storage area 322. The training data acquired via the data acquisition program 400 is input to the prediction model 410.

The loss calculation program 430 includes a command to execute processing of calculating a loss indicating an error between the predicted value of the stability label output from the prediction model 410 and the ground truth stability label. The optimizer 440 includes a command to execute processing of calculating an update amount of the parameter of the prediction model 410 from the calculated loss and updating the parameter of the prediction model 410. The optimizer 440 may optimize the parameter using, for example, a method such as stochastic gradient descent (SGD).

### <<Flowchart of Machine Learning Method>>

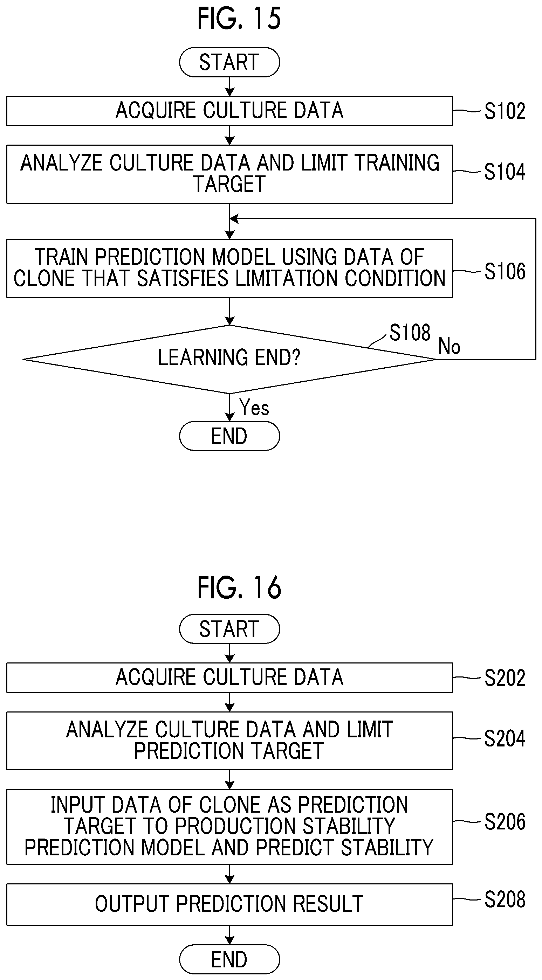

Fig. 15 is a flowchart showing an example of a machine learning method executed by the machine learning apparatus 300. Here, descriptions will be provided assuming that a dataset used for machine learning is prepared as illustrated in Figs. 6A and 6B. In step S102, the processor 302 acquires the culture data from the prepared dataset.

In step S104, the processor 302 analyzes the culture data and limits the training target. The processor 302 may sort whether the data is data of the target sample that satisfies the limitation condition or data of the non-target sample that does not satisfy the limitation condition in accordance with the index of the culture data and the threshold value designated in advance, or may search for the index and the threshold value as the limitation conditions based on the culture data and sort the data of the target sample and the data of the non-target sample.

In step S106, the processor 302 performs machine learning using only the data of the clone that satisfies the limitation condition to train the prediction model 410. That is, the processor 302 inputs the gene expression data of the sample that satisfies the limitation condition to the prediction model 410, and calculates a loss indicating an error between the predicted value of the stability label output from the prediction model 410 and the ground truth stability label. The processor 302 calculates an update amount of the parameter of the prediction model 410 based on the calculated loss and updates the parameter. In this way, the processor 302 trains the prediction model 410 such that the output (predicted value) from the prediction model 410 for the data input to the prediction model 410 approaches the ground truth stability label. The update of the parameter of the prediction model 410 may be carried out in mini-batch units.

In step S108, the processor 302 determines whether or not to end the learning. A learning end condition may be determined based on a value of the loss or may be determined based on the number of updates of the parameter. As a method based on the value of the loss, for example, the learning end condition may be that the loss converges within a prescribed range. Additionally, as a method based on the number of updates, for example, the learning end condition may be that the number of updates reaches a prescribed number of times. Alternatively, a dataset for performance evaluation of the model may be prepared separately from the training data, and whether or not to end the learning may be determined based on an evaluation value using the data for evaluation.

In a case in which the determination result in step S108 is No, the processor 302 returns to step S106 and continues the learning processing. On the other hand, in a case in which the determination result in step S108 is Yes, the processor 302 ends the flowchart in Fig. 12.

The trained prediction model 410 is incorporated into the information processing apparatus 10 as the production stability prediction model 16. The machine learning method executed by the machine learning apparatus 300 can be understood as a method of generating the production stability prediction model 16 and is an example of a prediction model generation method in the present disclosure.

### <<Flowchart of Information Processing Method of Predicting Production Stability>>

Fig. 16 is a flowchart showing an example of an information processing method executed by the information processing apparatus 10. In step S202, the processor 102 acquires the culture data measured for the clone that produces the useful substance. The processor 102 may automatically acquire data from a data storage server (not shown) or the like, or may receive an input of designation of data via a user interface and acquire data for the designated clone.

In step S204, the processor 102 analyzes the culture data and limits the prediction target. The processor 102 limits the prediction target by applying the same limitation conditions as the conditions in which the training target is limited in a case in which the production stability prediction model 16 is trained. By measuring the gene expression data for the clone corresponding to the prediction target after the prediction target is limited in step S204, it is possible to reduce the workload and the cost as compared to a case in which the genetic analysis of all the clones is carried out.

In step S206, the processor 102 inputs the gene expression data of the clone corresponding to the prediction target to the production stability prediction model 16, and the stability is predicted by the production stability prediction model 16.

In step S208, the processor 102 outputs the prediction result output from the production stability prediction model 16. The producing clone can be selected based on the prediction result of the production stability.

After step S208, the processor 102 ends the flowchart of Fig. 16.

### <<Regarding Program for Operating Computer>>

A program for causing a computer to implement some or all of the processing functions in each of the information processing apparatus 10 and the machine learning apparatus 300 is recorded on a computer-readable medium, which is a tangible non-transitory information storage medium such as an optical disc, a magnetic disc, or a semiconductor memory, and the program can be provided through the information storage medium.

In addition, instead of the aspect in which the program is stored in such a tangible non-transitory computer-readable medium and then provided, it is also possible to provide the program signals as a download service using a telecommunication line such as the Internet.

Further, some or all of the processing functions in each of the apparatuses mentioned above may be implemented by cloud computing or can also be provided as software as a service (SaaS).

### <<Regarding Hardware Configuration of Each Processing Unit>>

The hardware structure of the processing unit that executes various kinds of processing such as the data acquisition unit 12, the prediction target limitation unit 14, the stability prediction unit including the production stability prediction model 16, the processing result output unit 18, the culture data analysis section 20, the limitation condition determination section 22 in the information processing apparatus 10, the learning unit including the prediction model 410, the loss calculation unit, the parameter update amount calculation unit, and the parameter update unit in the machine learning apparatus 300 is, for example, various processors as shown below.

The various processors include a CPU, which is a general-purpose processor that executes programs and that functions as various processing units, a GPU, a programmable logic device (PLD), which is a processor whose circuit configuration can be changed after manufacturing such as a field programmable gate array (FPGA), dedicated electrical circuitry, which is a processor having a dedicated circuit configuration designed specifically to execute specific processing such as an application specific integrated circuit (ASIC), and the like.

A single processing unit may be composed of one of these various processors or two or more processors of the same type or different types. For example, a single processing unit may be composed of a plurality of FPGAs, a combination of a CPU and an FPGA, or a combination of a CPU and a GPU. Additionally, a plurality of processing units may be composed of a single processor. As an example of configuring a plurality of processing units with a single processor, first, there is an aspect in which a single processor is composed of one or more CPUs combined with software, as represented by computers such as clients or servers, and this processor functions as a plurality of processing units. Second, there is an aspect in which a processor that implements the functions of an entire system, including a plurality of processing units, with a single integrated circuit (IC) chip is used, as represented by systems on chip (SoC) and the like. In this manner, the various processing units are configured using one or more of the above-described various processors as a hardware structure.

Furthermore, the hardware structure of these various processors is, more specifically, electrical circuitry combined with circuit elements such as semiconductor elements.

### <<Advantage of Embodiment>>

With the method of predicting the production stability of the producing clone according to the above-mentioned embodiment and the information processing apparatus 10 that executes the method, the following effects can be obtained.

[1] Since the clone as the prediction target is appropriately limited based on the index of the culture data at the present point in time (at the time of the standard test), the production stability for the clone as the prediction target can be predicted with high accuracy.

[2] Since the genetic analysis (RNA-Seq analysis) need only be performed by limiting clones to the prediction target, it is possible to reduce the cost as compared to a case in which the genetic analysis is performed for all the clones.

[3] By applying the method according to the present embodiment instead of the stability test in the related art, it is possible to achieve a shortened development process for the producing cells and a reduction in cost.

### <<Others>>

The present disclosure is not limited to the above-mentioned embodiment, and various modifications are possible within the scope of the technical concept of the present disclosure without departing from its gist.

### Explanation of References

10: information processing apparatus
12: data acquisition unit
14: prediction target limitation unit
16: production stability prediction model
18: processing result output unit
20: culture data analysis section
22: limitation condition determination section
102: processor
104: computer-readable medium
106: communication interface
108: input/output interface
110: bus
112: memory
114: storage
120: data storage area
140: prediction target limitation program
142: culture data analysis program
144: limitation condition determination program
152: input device
154: display device
180: processing result output program
190: display control program
300: machine learning apparatus
302: processor
304: computer-readable medium
306: communication interface
308: input/output interface
310: bus
312: memory
314: storage
320: prediction target limitation program
322: prediction target data storage area
330: learning processing program
340: display control program
352: input device
354: display device
400: data acquisition program
410: prediction model
430: loss calculation program
440: optimizer
550: data storage unit
DSA, DSB: dataset
DSc, DSd, DSe: dataset
F5A: left diagram
F5B: right diagram
G1: graph
G2: graph
GEP: gene expression pattern
MLM: machine learning model
MLMc, MLMe: machine learning model
RF1 to RF7: rectangular frame
S102 to S108: steps of machine learning method
S202 to S208: steps of information processing method of predicting production stability

## Claims

1. A method of predicting production stability of a clone that produces a useful substance, the method comprising:
causing one or more processors to execute:
acquiring culture data of one or more types of clones;
analyzing the culture data and limiting the clones to a prediction target; and
using data measured for a clone as the prediction target to predict the production stability of the useful substance by the clone as the prediction target.

2. The method according to claim 1,
wherein the production stability is defined by presence or absence of a change in a production amount of the useful substance between a start of culture and after a predetermined culture period.

3. The method according to claim 1, further comprising:
causing the one or more processors to execute
setting an index obtained from the culture data and a threshold value related to the index,
wherein the prediction target is limited based on a value of the index and the threshold value.

4. The method according to claim 3,
wherein the threshold value is adjusted such that prediction accuracy of the production stability is higher than prediction accuracy in a case in which the prediction target is not limited.

5. The method according to claim 3,
wherein the threshold value is defined using a ranking of the value of the index.

6. The method according to claim 3,
wherein the prediction target is a top population of the values of the index.

7. The method according to any one of claims 3 to 6,
wherein the index is a production amount of the useful substance.

8. The method according to any one of claims 3 to 6,
wherein the index is an integral viable cell density.

9. The method according to any one of claims 3 to 6,
wherein the index is a lactic acid concentration.

10. The method according to any one of claims 1 to 6,
wherein the data used for the prediction of the production stability includes one or more gene expression levels.

11. The method according to any one of claims 1 to 6,
wherein the one or more processors
predict the production stability by using a model that receives an input of the data of the prediction target and that performs two-class classification into stable or unstable.

12. The method according to claim 11,
wherein the model is a model that has been trained through machine learning using a plurality of pieces of training data in which the data for a training clone, which is limited in the same manner as the clone as the prediction target, and a ground truth stability label are associated with each other.

13. The method according to claim 12,
wherein the plurality of pieces of training data include the training data for a plurality of types of clones that produce different useful substances, and
the one or more processors predict the production stability for a clone that produces a useful substance different from the useful substance used for the training of the model.

14. The method according to any one of claims 1 to 6,
wherein the useful substance is any of a protein, a peptide, or a virus that is a pharmaceutical raw material.

15. The method according to any one of claims 1 to 6,
wherein the useful substance is an antibody or an antibody-like protein.

16. The method according to any one of claims 1 to 6,
wherein the clone is a vertebrate-derived cell.

17. The method according to any one of claims 1 to 6,
wherein the clone is a mammalian-derived cell.

18. The method according to any one of claims 1 to 6,
wherein the clone is a CHO cell or a HEK cell.

19. An information processing apparatus comprising:
one or more processors; and
one or more storage devices that store a command to be executed by the one or more processors,
wherein the one or more processors
acquire culture data of one or more types of clones for a clone that produces a useful substance,
analyze the culture data and limit the clones to a prediction target, and
use data measured for a clone as the prediction target to predict production stability of the useful substance by the clone as the prediction target.

20. A program for causing a computer to implement:
a function of acquiring culture data of one or more types of clones for a clone that produces a useful substance;
a function of analyzing the culture data and limiting the clones to a prediction target; and
a function of using data measured for a clone as the prediction target to predict production stability of the useful substance by the clone as the prediction target.

21. A non-transitory computer-readable recording medium on which the program according to claim 20 is recorded.

22. A prediction model generation method of generating a prediction model that causes a computer to implement a function of predicting production stability of a clone that produces a useful substance, the prediction model generation method comprising:
causing a system including one or more processors to execute:
acquiring culture data of one or more types of clones;
analyzing the culture data and limiting the clones to a prediction target; and
performing machine learning using a plurality of pieces of training data in which data measured for a clone corresponding to the prediction target and a ground truth stability label are associated with each other, and training the prediction model such that an output of the prediction model in response to an input of the data approaches the ground truth stability label.
